(19) Europäisches Patentamt
European Patent Office
Office européen des brevets

(11) Veröffentlichungsnummer : **0 015 436**
**B1**

(12) # EUROPÄISCHE PATENTSCHRIFT

(45) Veröffentlichungstag der Patentschrift : **03.02.82**

(51) Int. Cl.³ : **C 07 C 50/14**, C 07 C 46/06, C 07 C 37/11, C 07 C 11/22

(21) Anmeldenummer : **80100822.8**

(22) Anmeldetag : **19.02.80**

(54) **Verfahren zur stereospezifischen Herstellung von Verbindungen der Vitamin K1- und K2-Reihe sowie neue Ausgangsprodukte in diesem Verfahren.**

(30) Priorität : **08.03.79 DE 2909091**
**15.01.80 CH 306/80**

(43) Veröffentlichungstag der Anmeldung : **17.09.80 (Patentblatt 80/19)**

(45) Bekanntmachung des Hinweises auf die Patenterteilung : **03.02.82 Patentblatt 82/05**

(84) Benannte Vertragsstaaten : **AT BE CH DE FR GB IT NL**

(56) Entgegenhaltungen : **Keine**

**ANGEWANDTE CHEMIE, Band 87, 1975 K.H. DÖTZ « Aufbau des Naphthol-gerüsts aus Pentacarbonyl-(methoxy(phenyl)carben) chrom (0) und Tolan » Seiten 672 bis 673**

**CHEMISCHE BERICHTE, Band 110, 1977 Weinheim K.H. DÖTZ et al. « Templat-Reaktionen an Chrom (0) Stereoselektive Synthese komplexgebundener substituierter Naphthaline » Seiten 1 555 bis 1 557**

**CHEMISCHE BERICHTE, Band 111, 1978, Weinheim K.H. DÖTZ et al. « Templat-Reaktionen an Chrom (0) : Stereoselektive Synthese kondensierter aromatischer Liganden aus Pentacarbonyl-Carben-Chrom-Komplexen und Alkinen » Seiten 2 517 bis 2 519**

**JOURNAL OF ORGANOMETALLIC CHEMISTRY, Band 140, 1977 Lausanne**

(73) Patentinhaber : **F. HOFFMANN-LA ROCHE & CO. Aktiengesellschaft**

**CH-4002 Basel (CH)**

(72) Erfinder : **Dötz, Karl Heinz, Dr.**
**Zeisigstrasse 30e**
**D-8011 Vaterstetten (DE)**

(74) Vertreter : **Hansen, Bernd, Dr.rer.nat. et al**
**Hoffmann . Eitle & Partner Patentanwälte Arabellastrasse 4**
**D-8000 München 81 (DE)**

(56) Entgegenhaltungen : **Keine**
**K.H. DÖTZ « Reaktionen von Komplexliganden. IX Carbenliganden, Carbonylliganden und Alkine als Bausteine für Isocyclische und Heterocyclische Systeme » Seiten 117 bis 179, 183 und 185 bis 186**

**EP 0 015 436 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur stereospezifischen Herstellung von Verbindungen der Vitamin $K_1$- und $K_2$-Reihe sowie neue Ausgangsprodukte in diesem Verfahren

Die Erfindung betrifft ein Verfahren zur stereospezifischen Herstellung von Verbindungen der Vitamin $K_1$- und $K_2$-Reihe der allgemeinen Formel

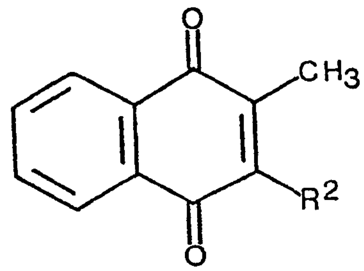

(IV)

worin

$R^2$ einen Dimethylallyl-, Geranyl-, Farnesyl- oder analogen isoprenoiden Rest bzw. den Phytyl-Rest darstellt.

Derartige Verbindungen sind, zum Teil als Naturstoffe, bekannt. Sie werden als Zusätze für Futterstoffe, für Untersuchungen von Metabolismen und andere Zwecke verwendet. Die genannten Substanzen können bezüglich der ersten Doppelbindung (vom Ringsystem aus gesehen) in der Terpenkette in Z-Form (auch Cis-Form genannt) oder in E-Form (auch Trans-Form genannt) oder als Gemisch dieser beiden Formen vorliegen. Bei biologischen Untersuchungen hat sich die Z-Form als biologisch weniger aktiv, wenn nicht sogar wirkungslos erwiesen. Bei Vitamin K wurde in J. Nutr. 105 : 1519-1524, 1975 für die Cis-Form ein « lack of biological activity » festgestellt. Nach Feststellungen von O. Isler in Angew. Chem., 71 (1959) Nr. 1, Seiten 13-15, zeigten bei Substanzen der Vitamin $K_1$- und $K_2$-Reihe die Mono-Cis-Verbindungen (Cis-Doppelbindung in der Nähe des Naphthochinon-Ringsystems) eine signifikant geringere Wirkung als die All-Trans-Formen.

Die Bemühungen um die synthetische Herstellung von Substanzen der Vitamin $K_1$- und $K_2$-Reihen — die K-Vitamine beeinflussen die Biosynthese des Prothrombins und anderer Blutgerinnungsfaktoren — waren schon früh erfolgreich. So wurde bereits 1939 ein Verfahren zur Herstellung von Vitamin $K_{1(20)}$, ausgehend von einem Methylnaphthohydrochinon und dem entsprechenden Terpenalkohol mit einem nachfolgenden Oxidationsschritt beschrieben. Hier wurde eine Gesamtausbeute von 29 %, bezogen auf Phytol, ohne Aufschlüsselung des erzielten E/Z-Verhältnisses angegeben.

Jüngere Verfahren, z.B. gemäss US-PS 2 683 176 bzw. DE-OS 2 733 233 gehen ebenfalls von entsprechenden methylsubstituierten Naphthohydrochinon-monoäther-Derivaten aus, auf die die entsprechenden Terpenalkohole bzw. Terpenäther aufkondensiert und das entstehende Reaktionsprodukt oxidiert werden. Während bei dem erstgenannten Verfahren z.B. eine Ausbeute an Vitamin $K_1$ von 37,5 %, bezogen auf Phytol, bei einem E/Z-Verhältnis von 90 bis 92,5/7,5 bis 10 erhalten wird, führt das letztgenannte Verfahren bei guter Ausbeute an Vitamin $K_1$ zu der E-Isomerenform.

Bei anderen Verfahren werden Methylnaphthohydrochinon und die entsprechenden Terpen-Derivate eingesetzt und in Gegenwart von Metallen, wie Zinkamalgam oder Zinkstaub, kondensiert. Hierbei entstehen zum Teil niedrige Gesamtausbeuten, wobei auch E/Z-Isomerengemische anfallen. Auch die Verwendung von N-Sulfinylaminen als Kondensationsmittel ist beschrieben worden, wobei sich, bezogen auf Phytol, Ausbeuten an Vitamin $K_1$ von nur 4 bis 7 %, wenn auch in Form des reinen E-Isomeren ergeben haben.

Es besteht somit ein Bedarf an der Schaffung von Verfahren zur Herstellung von Verbindungen der Vitamin $K_1$- und Vitamin $K_2$-Reihe, bei denen diese Verbindungen in hoher Ausbeute auf relativ einfache Weise anfallen. Da die Synthese der speziell methylsubstituierten Naphthohydrochinonderivate aufwendig ist, sind besonders solche Verfahren vorteilhaft, bei denen dieses Ausgangsprodukt nicht eingesetzt werden muss. Hierbei ist es insbesondere erwünscht, dass diese Verfahren stereospezifisch zu der ausschliesslich bzw. überwiegend biologisch aktiven E-Form führen.

Der Erfindung liegt die Aufgabe zugrunde, ein Verfahren zur stereospezifischen Herstellung von Verbindungen des Vitamin $K_1$- und Vitamin $K_2$-Typus zu schaffen, bei dem die genannten Verbindungen stereospezifisch in der E-Form in hoher Ausbeute anfallen, und das einfach durchführbar ist. Insbesondere soll das Verfahren in wenigen Verfahrensschritten unter einfacher intermediärer Ausbildung des Naphtholringes rasch ablaufen.

Diese Aufgabe wird nun durch das erfindungsgemässe Verfahren gelöst. Dieses Verfahren ist dadurch gekennzeichnet, dass man einen Phenyl-carben-carbonyl-Metallkomplex der Formel

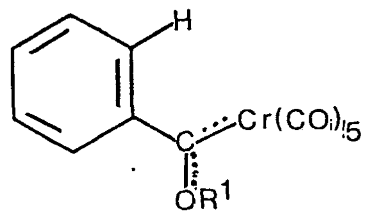

(I)

worin

$R^1$ niederes Alkyl, Acyl oder Benzyl darstellt, mit einem Enin der allgemeinen Formel

$$CH_3-C\equiv C-R^2 \qquad (II)$$

worin

$R^2$ einen Dimethylallyl-, Geranyl-, Farnesyl- oder analogen isoprenoiden Terpenylrest bzw. den Phytyl-Rest darstellt, umsetzt, in dem so erhaltenen substituierten Naphthol-Carbonyl-Metallkomplex der allgemeinen Formel

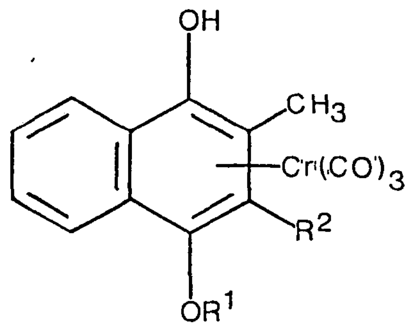

(III)

worin

$R^1$ und $R^2$ die obige Bedeutung haben, die Metall-Ring-Bindung spaltet und, erforderlichenfalls (d.h. falls die Spaltung nicht wie nachfolgend erwähnt direkt zum Endprodukt führt), das erhaltene Naphtholderivat oxidiert.

Durch dieses Verfahren sind die jeweils gewünschten Substanzen der Vitamin $K_1$- bzw. $K_2$-Reihe je nach Art des Substituenten $R^2$ in hoher Ausbeute zugängig, wobei die gewünschte E-Form überraschenderweise praktisch ausschliesslich anfällt, wenn das Ausgangs Enin ebenfalls in E-Konfiguration eingesetzt wird.

Im Rahmen der vorliegenden Erfindung bedeutet der Ausdruck « niederes Alkyl » Alkylgruppen mit 1-7 Kohlenstoffatomen, wie Methyl, Aethyl, Propyl, Isopropyl und dergleichen, wobei Methyl bevorzugt ist. Der Ausdruck « Acyl » bedeutet niederes Alkanoyl mit bis zu 7 Kohlenstoffatomen wie z.B. Acetyl, Propionyl, Butyryl und dergleichen oder auch Aroyl wie z.B. Benzoyl usw.

Die als Ausgangsprodukte verwendeten Phenyl-carben-carbonyl-Metallkomplexe sind bekannt oder Analoge bekannter Verbindungen und können z.B. gemäss Darensbourg et al., Inorg. Chem. 9, 32 (1970) hergestellt werden. Für die Zwecke der vorliegenden Erfindung besonders bevorzugt sind der Pentacarbonyl (methoxy-phenylcarben) chrom-Komplex sowie der Pentacarbonyl (acetoxy-phenylcarben) chrom-Komplex. Sämtliche obigen Komplexe stellen Verbindungen dar, die unter $N_2$-Schutz und bei tiefen Temperaturen hohe Lagerstabilität aufweisen.

Die als weitere Reaktionspartner dienenden Enine der allgemeinen Formel II sind zum Teil bekannte und zum Teil neue Verbindungen. Die neuen Verbindungen können in zur Herstellung der bekannten analoger Weise hergestellt werden und sind ebenfalls Gegenstand der vorliegenden Erfindung. Bekannt sind beispielsweise das 5-Methyl-4-hexen-2-in sowie das 6-Methyl-5-hepten-2-in und die neuen Geranyl-, Farnesyl- bzw. Phytyl-Derivate, nämlich 6.10-Dimethyl-5.9-undecadien-2-in ; 6.10.14-Trimethyl-5.9.13-pentadecatrien-2-in bzw. 6.10.14.18-Tetramethyl-5-nonadecen-2-in lassen sich auf analoge Weise leicht synthetisieren. Im allgemeinen geht man hierbei von Propinylmagnesiumbromid in Aether aus und tropft eine Lösung des jeweiligen Terpenhalogenides, insbesondere Terpenbromides R-Br (R = Geranyl, Farnesyl, Phytyl) in Aether langsam zu. Nach mehrstündigem Erwärmen unter Rückfluss giesst man auf Eis und gegebenenfalls verdünnte Säure (z.B. Essigsäure) und extrahiert mit organischem Lösungsmittel. Nach Waschen und üblicher Aufarbeitung erhält man dann das gewünschte Enin als Rohprodukt in guter Ausbeute.

Die Umsetzung des Enins der Formel II (bei dessen Verwendung in der E-Form) mit dem Metall-Carben-Komplex der Formel I führt in einstufiger, schnell ablaufender Reaktion direkt und stereospezifisch zu dem E-Typus des jeweils gewünschten Vitamin K-Produktes in der hydrochinoiden Form der Formel III, die dann unter Erhalt der gewünschten E-Form, in das gewünschte Endprodukt der allgemeinen Formel

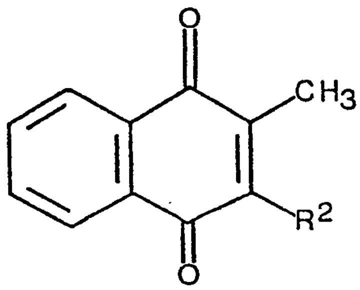

(IV)

worin

$R^2$ die obige Bedeutung hat, übergeführt werden kann.

Bei dem erfindungsgemässen Verfahren wird die Umsetzung des Carben-Komplexes der Formel I mit dem Enin der Formel II zweckmässig unter Schutzgasatmosphäre, z.B. unter Stickstoff, Argon, etc., durchgeführt. Es kann jedoch auch ein Sauerstoffzutritt auf andere geeignete Weise vermieden werden.

Die Reaktion läuft zweckmässig in einem Donorlösungsmittel ab, wobei Aether besonders bevorzugt sind. Hierbei lassen sich mit Vorteil höher siedende Aether einsetzen, z.B. tert.-Butylmethyläther oder Dibutyläther, wobei dann die Reaktion bei erhöhter Temperatur bequem ablaufen kann.

Wenngleich die Reaktion bereits bei Raumtemperatur abläuft, ist es bevorzugt, die Umsetzung z.B. bei einer Temperatur im Bereich zwischen etwa 25° und etwa 80 °C und insbesondere zwischen etwa 50° und etwa 60 °C durchzuführen. Die Reaktion läuft bei stöchiometrischen Verhältnissen der Reaktionspartner gut und vollständig ab, wobei die substituierten Naphthol-tricarbonyl-chrom-Komplexe in Ausbeuten von mindestens 85 bis zu 100 % der Theorie anfallen. Vorzugsweise wird jedoch der Anteil an Enin gegenüber der Ausgangscarbenverbindung in schwach überstöchiometrischem Verhältnis, beispielsweise 1,1 : 1 = Enin : Ausgangscarben-Komplex, eingesetzt.

Die Naphthol-tricarbonyl-chrom-Komplexe der Formel III sind neue Verbindungen und ebenfalls Gegenstand der vorliegenden Erfindung.

Die Spaltung der Metall-Ring-Bindung in den Verbindungen der Formel III kann auf verschiedene Art und Weise durchgeführt werden. So kann diese Spaltung z.B. mit Hilfe eines Oxidationsmittels durchgeführt werden, wobei dann auch gleichzeitig die Oxidation des Naphtholderivates zur Verbindung der Formel IV erfolgt. Diese Reaktion lässt sich im direkten Anschluss an die Bildung des substituierten Naphthol-carbonyl-Metallkomplexes der Formel III im Eintopfverfahren oder auch nach Isolierung des Komplexes der Formel III und dessen Reinigung, sofern dies zweckmässig erscheint, durchführen.

Als Oxidationsmittel kann Silberoxid dienen, das den Terpensubstituenten intakt lässt, jedoch zur Spaltung der Metall-Ring-Bindung in der Lage ist und die Hydrochinon-Oxidationsstufe leicht zu der chinoiden Endform überführen kann. Bei der oxidativen Spaltung der Metall-Ring-Bindung mit $Ag_2O$ kann jedoch neben dem gewünschten Chinon auch eine geringe Menge (z.B. 5 %) des Chinon-$Cr(CO)_3$-Komplexes der Formel

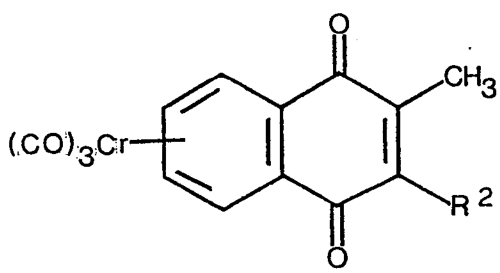

(V)

worin

$R^2$ die obige Bedeutung hat, anfallen. Das Auftreten dieses unerwünschten Nebenproduktes lässt sich jedoch durch die Verwendung starker Oxidationsmittel, wie z.B. $H_2O_2$, $MnO_2$, $PbO_2$ oder $NiO_2$ und dergleichen, verhindern. Es ist daher bei manchen Ausführungsformen der Erfindung bevorzugt, diese Reaktion beispielsweise mit 20 %-igem $H_2O_2$, z.B. in einem unpolaren Lösungsmittel bei Raumtemperatur, durchzuführen.

Wird die Oxidation direkt anschliessend an die Bildung des Komplexes der Formel III ohne dessen Isolierung durchgeführt, kann die Oxidation zweckmässig durch Silber(I) oxid in Gegenwart von $MgSO_4$ oder anderen wasserentziehenden Mitteln durchgeführt werden.

Ferner kann die Spaltung der Metall-Ring-Bindung in den Verbindungen der Formel III im Prinzip auch mit all denjenigen Reagenzien durchgeführt werden, mit denen man üblicherweise niedere Oxidationsstufen von Uebergangselementen stabilisieren kann. Als Beispiele derartiger Reagenzien können genannt werden : Kohlenmonoxid, welches vorzugsweise unter Druck, insbesondere unter einem Druck von etwa 50 bis etwa 100 Atmosphären angewandt wird, Phosphine, wie z.B. Triphenylphosphin, Phosphite, wie z.B. Trimethylphosphit, Isonitrile, Olefine, wie z.B. Cyclooctadien oder Norbornadien, Aromaten, wie z.B. Benzol, Methyl- oder Halogenbenzole, Benzoesäureester, Anilin bzw. Alkylderivate

hiervon, oder auch Stickstoffbasen, wie z.B. Pyridin und dergleichen.

Erfolgt die Spaltung der Metall-Ring-Bindung nicht oxidativ, sondern gemäss einer der anderen vorhergehend erwähnten Methoden, so muss anschliessend das erhaltene Naphtholderivat noch zur Verbindung der allgemeinen Formel IV oxidiert werden. Diese Oxidation kann in an sich bekannter Weise durchgeführt werden, z.B. mittels Silberoxyd oder auch mittels Luftsauerstoff.

Das bei dieser Methode anfallende Chromcarbonyl kann, gegebenfalls nach Behandlung mit Kohlenmonoxid, als Chromhexacarbonyl wieder in den Prozess zurückgeführt werden.

Der prinzipielle Ablauf des erfindungsgemässen Verfahrens wird nachstehend anhand einzelner Arbeitsvorschriften für die Herstellung der Enine, soweit sie nicht vorbekannt sind, der Herstellung des Reaktionskomplexes der Formel III, sowie der Spaltung der Metall-Ring-Bindung und der Oxidationsstufe, die zu den Endprodukten der Formel IV führt, charakterisiert.

## Beispiel 1

### Herstellung der Enine der Formel II

Zu einer Lösung von 30 mmol Propinylmagnesiumbromid in 20 ml Aether gibt man eine Spatelspitze Kupfer(I) chlorid und lässt anschliessend eine Lösung von 30 mmol R-Br (R = Geranyl, Farnesyl, Phytyl) in 20 ml Aether langsam zutropfen. Nach zwölfstündigem Erwärmen unter Rückfluss giesst man auf Eis und verdünnte Essigsäure und extrahiert mit Aether. Man wäscht mit verdünnter Natronlauge und Wasser neutral und trocknet über $MgSO_4$. Nach dem Abziehen des Lösungsmittels erhält man das Enin als Rohprodukt in 65 bis 85 % Ausbeute.

Trans-(10R,14R)-6,10,14,18-Tetramethyl-5-nonadecen-2-in : Siedepunkt 131°-145 °C/0,15 mmHg

## Beispiel 2

### Herstellung der Verbindungen der Formel III

1 mmol Carbonyl-carben-chrom-Komplex wird unter Rühren mit 1,1 mmol Enin in 5 ml eines Donorlösungsmittels unter Schutzgas ($N_2$) 1/2 bis 3 Stunden auf 25° bis 80 °C erwärmt. Man erhält nach Chromatographie über Kieselgel mit Methylenchlorid/Pentan-Gemischen die substituierten Naphthol-tricarbonyl-chrom-Komplexe der Formel III in Ausbeuten von 85 bis 100 %.

## Beispiel 3

Spaltung der Metall-Ring-Bindung und gleichzeitige Oxidation des erhaltenen Naphtholderivates (hier nach vorhergehender Isolierung des erhaltenen Komplexes der Formel III).

Der aus 1 mmol Carbonyl-carben-chrom-Komplex und 1,1 mmol rohem Enin erhaltene 2.3.4-trisubstituierte 1-Naphtholtricarbonyl-chrom-Komplex wird in Aether mit einem Ueberschuss an $Ag_2O$ oxidiert. Die chromatographische Aufarbeitung an Kieselgel mit Pentan/Aether-Gemischen bei − 10° bis 20 °C liefert 30 bis 50 % 2.3-disubstituiertes Naphthochinon.

Der Ansatz kann proportional vergrössert werden.

## Beispiel 4

Spaltung der Metall-Ring-Bindung mittels Kohlenmonoxid.

Der aus 1 mmol Carbonyl-carben-chrom-Komplex und 1,1 mmol rohem Enin erhaltene 2.3.4-trisubstituierte 1-Naphthol-tricarbonyl-chrom-Komplex wird in Aether in einem Stahlautoklaven unter einem Kohlenmonoxid-Druck von etwa 50 bis etwa 100 at. auf ca. 60° bis 100 °C erwärmt. Nach Entspannen des Autoklaven wird die Lösung filtriert, das Lösungsmittel aus dem Filtrat entfernt und der Rückstand chromatographiert, wobei man das gewünschte Naphtholderivat erhält, welches durch anschliessende Oxidation in das Endprodukt der Formel IV übergeführt werden kann.

Die in den obigen Beispielen genannten Arbeitsbedingungen können entsprechend abgeändert werden, da sie lediglich eine bevorzugte Ausführungsform der Erfindung darstellen, die nicht einschränkend aufzufassen ist.

Nachstehend soll nun die Herstellung von Vitamin $K_{1(20)}$ beschrieben werden.

## Beispiel 5

### Herstellung von Vitamin $K_1$

Eine Lösung von 1 mmol Pentacarbonyl [methoxy(phenyl) carben]-chrom und 1,1 mmol 6.10.14.18-Tetramethyl-5-nonadecen-2-in aus Phytylbromid (Isomerenverhältnis E/Z = 90/10) in 5 ml Dibutyläther wird unter Stickstoff 1 Stunde auf 55 °C erwärmt. Nach dem Abziehen des Lösungsmittels kann der 1-Naphthol-tricarbonyl-chrom-Komplex durch Chromatographie an Kieselgel bei − 30 °C mit Methylenchlorid/Pentan (2/1) isoliert (Ausbeute 95 %) oder direkt nach Zugabe von 10 ml Aether durch einstündiges Rühren mit 1,5 mmol Silber(I) oxid in Gegenwart von $MgSO_4$ zum Chinon oxidiert werden. Man filtriert, entfernt das Lösungsmittel und reinigt durch Chromatographie bei 10 °C an Kieselgel mit Pentan/Aether (100/l). Man erhält Vitamin $K_{1(20)}$ als hellgelbes Oel in einer Gesamtausbeute von 56 %.

Isomerenverhältnis E/Z = 87 : 13 ( ± 5) ($^1$H-NMR-spektroskopisch bestimmt).

Das beobachtete Isomerenverhältnis E/Z war bereits (innerhalb der Fehlergrenzen) in dem Ausgangs-Enin vorhanden, wodurch der Nachweis eines streng stereospezifischen Ablaufes der beschriebenen Additionsreaktion erbracht ist.

Die erhaltenen Endprodukte wurden durch IR-, NMR-Spektren sowie massenspektroskopische Untersuchungen durch Vergleich mit bekannten Spektren identifiziert. Auch wurden die Reinheitsbestimmungen sowie die Feststellung des E/Z-Isomerenverhältnisses aufgrund der $^1$H-NMR-Spektroskopie und der CH-Analyse durchgeführt.

Naschstehend sind die für Verbindungen der $K_1$- und $K_2$-Reihe erhaltenen Analysenwerte sowie die Isomerenverhältnisse E/Z wie folgt angegeben.

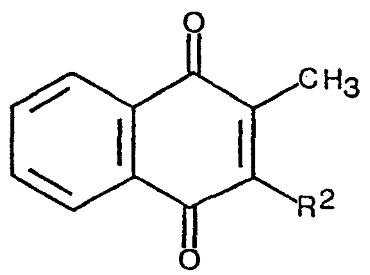

(IV)

| | E/Z | Analysenwerte | Ausbeute (bezogen auf R-Br) |
|---|---|---|---|
| $R^2$ = Diméthylallyl 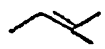 | — | ber. : C 79,97 H 6,71<br>gef. : C 80,42 H 7,35 | 51 % |
| $R^2$ = Geranyl ($K_{2(10)}$) 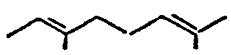 | 85 : 15 | ber. : C 81,78 H 7,84<br>gef. : C 81,47 H 8,20 | 54 % |
| $R^2$ = Farnesyl ($K_{2(15)}$) 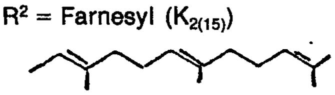 | 82 : 15 | ber. : C 82,93 H 8,57<br>gef. : C 82,88 H 8,97 | 55 % |
| $R^2$ = Phytyl ($K_{1(20)}$) 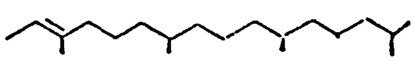 | 87 : 13 | ber. : C 82,61 H 10,29<br>gef. : C 82,29 H 10,63 | 56 % |

Die obigen Werte zeigen die erreichten hohen Ausbeuten an. Die Ausbeuten sind auf hochgereinigte Produkte bezogen und wurden nicht optimiert. Gleichzeitig wird ersichtlich, dass die Reaktion streng stereospezifisch im Hinblick auf die alleinige Ausbildung der gewünschten biologisch aktiveren Form abläuft. Dies wurde dadurch verifiziert, dass das gewählte E/Z-Verhältnis der eingesetzten Enine (Geranylbromid, Farnesylbromid und Phytylbromid mit einem E/Z-Verhältnis von 85 : 15, 85 : 15 und 87 : 13) im Endprodukt der Synthese erhalten blieb. Beim Einsatz einheitlicher E-Formen des Enins ergeben sich daher ausschliesslich die gewünschten biologisch aktiveren Substanzen der Vitamin $K_1$- bzw. $K_2$-Reihe in der E-Form.

## Beispiel 6

Eine Lösung von 340 mg des Tricarbonyl (4-methoxy-2,3-methyl-phytyl-1-naphthol) chrom-Komplexes (hergestellt gemäss Beispiel 5) in 35 ml Ether wird in einem 100 ml — Stahlautoklaven unter einem Kohlenmonoxid-Druck von 85 at 6 Stunden auf 80 °C erwärmt. Nach dem Entspannen filtriert man die gelbe Lösung über Filterflocken und kühlt auf – 40 °C ab, wobei die Hauptmenge des entstandenen Hexacarbonylchroms ausfällt und bereits abgetrennt werden kann. Nach dem Abziehen des Lösungsmittels bleibt ein oranges Oel zurück, das zur weiteren Reinigung an Kieselgel chromatographiert wird. Das so erhaltene Naphtholderivat kann in bekannter Weise in Vitamin $K_1$ übergeführt werden.

**Ansprüche**

1. Verfahren zur stereospezifischen Herstellung von Verbindungen der Vitamin $K_1$- und $K_2$-Reihe der allgemeinen Formel

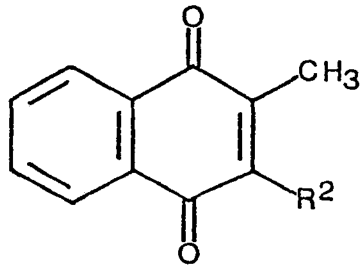

(IV)

worin

$R_2$ einen Dimethylallyl-, Geranyl-, Farnesyl- oder analogen isoprenoiden Rest bzw. den Phytyl-Rest darstellt, dadurch gekennzeichnet, dass man einen Phenyl-carben-carbonyl-metallkomplex der allgemeinen Formel

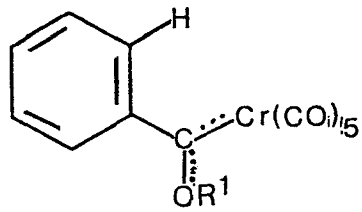

(I)

worin

$R^1$ niederes Alkyl, Acyl oder Benzyl darstellt, mit einem Enin der allgemeinen Formel

$$CH_3-C\equiv C-R^2 \qquad (II)$$

worin

$R^2$ die vorstehend angeführte Bedeutung besitzt, umsetzt, in dem so erhaltenen substituierten Naphthol-Carbonyl-Metallkomplex der allgemeinen Formel

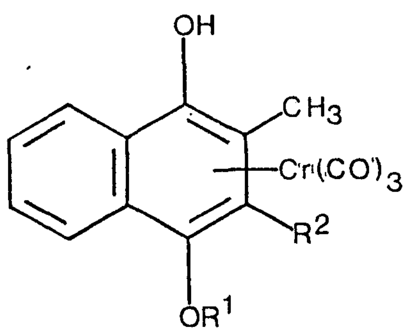

(III)

worin

$R^1$ und $R^2$ die obige Bedeutung haben, die Metall-Ring-Bindung spaltet und, erforderlichenfalls, das erhaltene Naphtholderivat oxidiert.

2. Verfahren gemäss Anspruch 1, dadurch gekennzeichnet, dass man das Enin der Formel II in Form des praktisch reinen E-Isomeren einsetzt.

3. Verfahren nach Anspruch 1 oder 2, dadurch gekennzeichnet, dass man als Phenyl-carben-carbonyl-Metallkomplex der Formel I eine Verbindung verwendet, worin $R^1$ die Methylgruppe oder die Acetylgruppe darstellt.

4. Verfahren nach Anspruch 1, 2 oder 3, dadurch gekennzeichnet, dass man die Umsetzung des Komplexes der Formel I mit dem Enin der Formel II unter Schutzgasatmosphäre durchführt.

5. Verfahren nach einem der Ansprüche 1 bis 4, dadurch gekennzeichnet, dass man die Umsetzung in einem Donorlösungsmittel unter Schutzgasatmosphäre durchführt.

6. Verfahren nach einem der Ansprüche 1 bis 5, dadurch gekennzeichnet, dass man das Enin der Formel II mit dem Phenyl-carben-carbonyl-Metallkomplex der Formel I im Verhältnis von 1,1 : 1, bezogen

auf Moläquivalente, umsetzt.

7. Verfahren nach einem der Ansprüche 1 bis 6, dadurch gekennzeichnet, dass man die Reaktion bei einer Temperatur zwischen 25 und 80 °C durchführt.

8. Verfahren nach Anspruch 7, dadurch gekennzeichnet, dass man die Reaktion bei 50 bis 60 °C in tert-Butylmethyläther bzw. Dibutyläther während einer Stunde durchführt.

9. Verfahren nach einem der Ansprüche 1 bis 8, dadurch gekennzeichnet, dass man die Spaltung der Metall-Ring-Bindung mit einem Oxidationsmittel durchführt.

10. Verfahren nach Anspruch 9, dadurch gekennzeichnet, dass man als Oxidationsmittel Silberoxid verwendet.

11. Verfahren nach Anspruch 9, dadurch gekennzeichnet, dass man als Oxidationsmittel $H_2O_2$, $MnO_2$ oder $NiO_2$ einsetzt.

12. Verfahren nach einem der Ansprüche 1 bis 8, dadurch gekennzeichnet, dass man als Reagenz zur Spaltung der Metall-Ring-Bindung Kohlenmonoxid, ein Phosphin, ein Phosphit, ein Isonitril, ein Olefin, einen Aromaten oder eine Stickstoffbase verwendet.

13. Verbindungen der allgemeinen Formel

$$CH_3-C\equiv C-R^2 \qquad (II)$$

worin

$R^2$ einen Geranyl-, Farnesyl- oder analogen isoprenoiden Terpenylrest bzw. den Phytyl-Rest darstellt.

14. Trans-(10R,14R)-6,10,14,18-Tetramethyl-5-nonadecen-2-in.

## Claims

1. A process for the stereospecific manufacture of compounds of the vitamin $K_1$ and $K_2$ series of the general formula

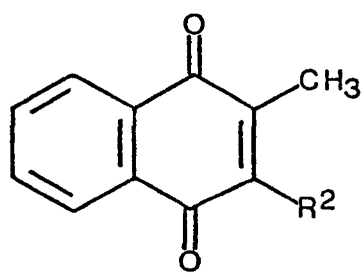

(IV)

wherein

$R^2$ represents a dimethylallyl, geranyl, farnesyl or analogous isoprenoid residue or the phytyl residue, characterized by reacting a phenyl-carbene-carbonyl-metal complex of the general formula

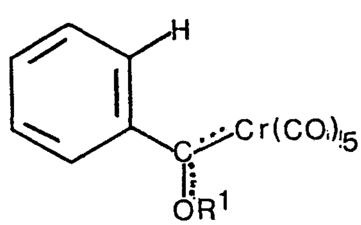

(I)

wherein

$R^1$ represents lower alkyl, acyl or benzyl, with an enyne of the general formula

$$CH_3-C\equiv C-R^2 \qquad (II)$$

wherein

$R^2$ has previously given significance, cleaving the metal-ring bond in the thus-obtained substituted naphthol-carbonyl-metal complex of the general formula

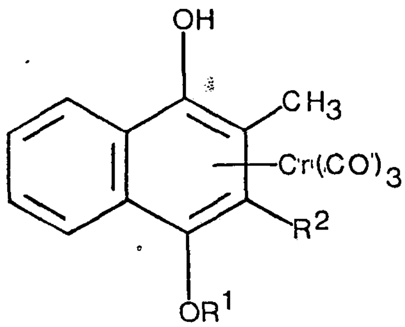

(III)

wherein

$R^1$ and $R^2$ have the above significance, and, if required, oxidizing the naphthol derivative obtained.

2. A process in accordance with claim 1, characterized in that the enyne of formula II is used in the form of the practically pure E-isomer.

3. A process according to claim 1 or 2, characterized in that a compound wherein $R^1$ represents the methyl group or the acetyl group is used as the phenyl-carbene-carbonyl-metal complex of formula I.

4. A process according to claim 1, 2 or 3, characterized in that the reaction of the complex of formula I with the enyne of formula II is carried out under a protective gas atmosphere.

5. A process according to any one of claims 1 to 4, characterized in that the reaction is carried out in a donor solvent under a protective gas atmosphere.

6. A process according to any one of claims 1 to 5, characterized in that the enyne of formula II is reacted with the phenyl-carbene-carbonyl-metal complex of formula I in the ratio of 1.1 : 1, based on molar equivalents.

7. A process according to any one of claims 1 to 6, characterized in that the reaction is carried out at a temperature between 25 and 80 °C.

8. A process according to claim 7, characterized in that the reaction is carried out at 50 to 60 °C in tert.-butyl methyl ether or dibutyl ether for one hour.

9. A process according to any one of claims 1 to 8, characterized in that the cleavage of the metal-ring bond is carried out with an oxidation agent.

10. A process according to claim 9, characterized in that silver oxide is used as the oxidation agent.

11. A process according to claim 9, characterized in that $H_2O_2$, $MnO_2$ or $NiO_2$ is used as the oxidation agent.

12. A process according to any one of claims 1 to 8, characterized in that carbon monoxide, a phosphine, a phosphite, an isonitrile, an olefin, an aromatic compound or a nitrogen base is used as the reagent for the cleavage of the metal-ring bond.

13. Compounds of the general formula

$$CH_3-C\equiv C-R^2 \qquad \text{(II)}$$

wherein

$R^2$ represents a geranyl, farnesyl or analogous isoprenoid terpenyl residue or the phytyl residue.

14. Trans-(10R,14R)-6,10,14,18-tetramethyl-5-nonadecen-2-yne.

**Revendications**

1. Procédé de préparation stéréospécifique de composés de la série des vitamines $K_1$ et $K_2$ de formule générale

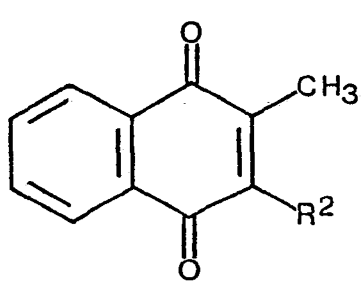

(IV)

où

$R^2$ représente un radical diméthylallyle, géranyle, farnésyle ou un radical isoprénoïde analogue ou le

radical phytyle, caractérisé en ce qu'on fait réagir un complexe phényl-carbène-carbonyl-métal de formule générale

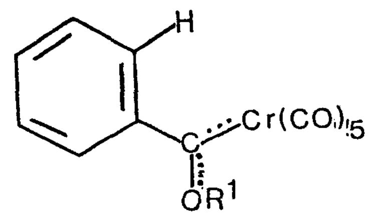

(I)

où

$R^1$ représente un alcoyle inférieur, un acyle ou un benzyle, avec une énine de formule générale

$$CH_3-C\equiv C-R^2 \quad (II)$$

où

$R^2$ possède la signification donnée ci-dessus, en ce que, dans le complexe naphtol-carbonyl-métal substitué ainsi obtenu de formule générale

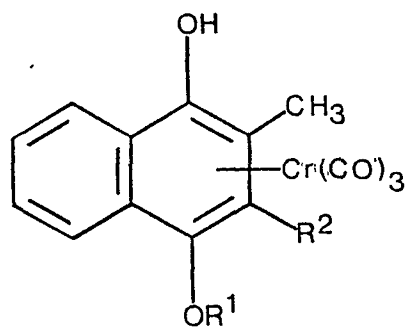

(III)

où

$R^1$ et $R^2$ ont la signification donnée ci-dessus, on coupe la liaison métal-noyau et, si nécessaire, on oxyde le dérivé naphtol obtenu.

2. Procédé selon la revendication 1, caractérisé en ce qu'on utilise l'énine de formule II sous la forme de l'isomère E pratiquement pur.

3. Procédé selon la revendication 1 ou 2, caractérisé en ce qu'on utilise comme complexe phényl-carbène-carbonyl-métal de formule I un composé où $R^1$ représente le groupe méthyle ou le groupe acétyle.

4. Procédé selon la revendication 1, 2 ou 3, caractérisé en ce qu'on conduit la réaction du complexe de formule I avec l'énine de formule II sous une atmosphère de gaz protecteur.

5. Procédé selon l'une des revendications 1 à 4, caractérisé en ce qu'on conduit la réaction dans un solvant donneur sous une atmosphère de gaz protecteur.

6. Procédé selon l'une des revendications 1 à 5, caractérisé en ce qu'on fait réagir l'énine de formule II avec le complexe phényl-carbène-carbonyl-métal de formule I dans un rapport de 1,1 : 1, en équivalents molaires.

7. Procédé selon l'une des revendications 1 à 6, caractérisé en ce qu'on conduit la réaction à une température comprise entre 25 et 80 °C.

8. Procédé selon la revendication 7, caractérisé en ce qu'on conduit la réaction à 50 à 60 °C dans le tert-butylméthyléther ou le dibutyl-éther pendant 1 heure.

9. Procédé selon l'une des revendications 1 à 8, caractérisé en ce qu'on effectue la coupure de la liaison métal-noyau avec un oxydant.

10. Procédé selon la revendication 9, caractérisé en ce qu'on utilise comme oxydant l'oxyde d'argent.

11. Procédé selon la revendication 9, caractérisé en ce qu'on utilise comme oxydant $H_2O_2$, $MnO_2$ ou $NiO_2$.

12. Procédé selon l'une des revendications 1 à 8, caractérisé en ce qu'on utilise comme réactif pour couper la liaison métal-noyau le monoxyde de carbone, une phosphine, un phosphite, un isonitrile, une oléfine, un aromatique ou une base azotée.

13. Composés de formule générale

$$CH_3-C \equiv C-R^2 \qquad (II)$$

où

$R^2$ représente un radical géranyle, farnésyle ou un radical terpényle isoprénoïde analogue ou le radical phytyle.

14. Trans-(10R,14R)-6,10,14,18-tétraméthyl-5-nonadécèn-2-ine.